# EUROPEAN PATENT APPLICATION

(11) **EP 2 671 944 A1**
(43) Date of publication of application: **11.12.2013**
(21) Application number: 12742430.7
(22) Date of filing: 30.01.2012
(51) Int. Cl.: C12N 5/07, C12N 5/073, C12Q 1/02

(54) **HIGHLY FUNCTIONAL LIVER CELLS DERIVED FROM PLURIPOTENT STEM CELLS, METHOD FOR PRODUCING SAME, AND METHOD FOR TESTING METABOLISM/TOXICITY OF DRUG**

(30) Priority: 31.01.2011 JP 2011019103; 05.08.2011 JP 2011171475
(71) Applicant: National Center for Global Health and Medicine, Tokyo 162-8655 (JP); Dnavec Corporation, Ibaraki 300-2611 (JP)
(72) Inventor: YUO, Akira, Tokyo 162-8655 (JP); SAEKI, Kumiko, Tokyo 162-8655 (JP); NAKAMURA, Naoko, Tokyo 162-8655 (JP); MATSUYAMA, Satoko, Tokyo 162-8655 (JP); NISHIO, Miwako, Tokyo 162-8655 (JP); SAEKI, Koichi, Tsukuba-shi Ibaraki 300-2611 (JP); HASEGAWA, Mamoru, Tsukuba-shi Ibaraki 300-2611 (JP)
(74) Representative: Rudolph, Ulrike
(86) International application number: PCT/JP2012/052007
(87) International publication number: WO 2012/105505

(57) **Abstract**

Functional hepatocytes that can be used for testing metabolism and toxicity of a drug are produced from pluripotent stem cells. A method of producing highly functional hepatocytes using pluripotent stem cells by preparing pluripotent stem cell-derived primitive endoderms from pluripotent stem cells by a method including the steps (A) and (B); preparing liver progenitor cells from the pluripotent stem cell-derived primitive endoderms by a method including the step (C); and preparing highly functional hepatocytes from the liver progenitor cells by a method including the step (D),
(A) culture under serum-free and feeder-free conditions;
(B) culture in the presence of albumin and at least one cytokine;
(C) culture in the presence of SHH or an SHH agonist and at least one cytokine; and
(D) culture to promote maturity in the presence of at least one cytokine.

## Description

### Technical Field

The present invention relates to a method of producing highly functional hepatocytes from pluripotent stem cells; a method of producing primitive endoderm and liver progenitor cells derived from pluripotent stem cells as intermediate products; primitive endoderms, liver progenitor cells, and highly functional hepatocytes derived from the pluripotent stem cells produced by the methods; and a method of testing metabolism and toxicity of drugs using the highly functional hepatocytes.

### Background Art

The liver is the body's major detoxification organ and participates in the metabolism of various drugs. It is known that every drug imposes a burden on the liver to a greater or less extent when the drug is administered to a human. The degree of the burden ranges widely from a slight changes in liver functions to severe liver failure, but there is no method that can appropriately estimate the degree in advance.

The verification of safety of a newly developed drug is currently performed through a clinical test (clinical trial), which is performed for obtaining an approval under the Pharmaceutical Affairs Law. The implementation of clinical trial, however, requires large amounts of budget and time. Another problem is that, recently, the ratio of drugs under research and development, of which development is discontinued due to hepatotoxicity thereof, has been increasing. In addition, some of the drugs that have passed the clinical trial and have been marketed are banned from being sold because of reports on cases of developing severe liver damage, and pharmaceutical companies also bear responsibility for the payment of compensation. For example, an antidiabetic, Rezulin, which was developed by a global mega pharmaceutical company, Pfizer Inc., was banned from being sold because of its hepatotoxicity. It has been reported this caused a loss of several hundred million dollars to Pfizer Inc. (Non-Patent Literature 1).

The hepatotoxicity of a drug largely varies depending on individuals and, therefore, may not be sufficiently comprehended during the clinical trial. Consequently, not a small number of drugs that have been launched cause unfortunate accidents and are thereby banned from being sold.

Accordingly, it is urgently necessary for drug discovery business to construct a system that can evaluate the metabolism and toxicity of a drug in advance by an in vitro test before implementation of the clinical trial. The provision of such a system highly contributes to improvement of drug discovery research and acceleration of drug discovery business at global levels.

In techniques currently used for *in vitro* testing of drug metabolism/toxicity, 1) human liver slices, 2) human primary cultured hepatocytes, 3) human hepatocyte cell lines, or 4) human hepatocyte microsomes are used.

Among them, human liver slices and human primary cultured hepatocytes significantly vary in their activities depending on lots and individuals and also show very unstable activities in *in vitro* experiments. Thus, the uses thereof involve serious problems.

Regarding human hepatocyte cell lines, it is known that many of them are unsuitable for being used in drug metabolism/toxicity testing. HapaRG (Non-Patent Literature 2) is the only one that is permitted to be used in drug metabolism/toxicity testing. However, it is a cell line derived from a specific individual, and therefore it is impossible to evaluate individual difference in drug metabolism/toxicity.

Human hepatocyte microsomes can be used in evaluation of drug metabolism. However, they are not hepatocytes themselves but materials merely purified from a microsome fraction. Therefore, they cannot be used for evaluating toxicity on hepatocytes themselves. The huge cost needed for preparing human hepatocyte microsomes is also a problem.

From these viewpoints, development of a technology for preparing functional hepatocytes from human pluripotent stem cells having both infinite proliferation ability and pluripotent differentiation ability can solve all the problems described above. That is, such a technology dramatically improves the quality and efficiency in evaluation of drug metabolism/toxicity.

There have been some reports on technologies for preparing functional hepatocytes from human embryonic stem (ES) cells or human induced pluripotent stem (iPS) cells, which are human pluripotent stem cells, and related technologies have also been reported (Patent Literatures 1 to 3).
Technologies that can detect the activity of a cytochrome P450 enzyme mainly involved in drug metabolism in the liver have also been reported. However, conventional technologies all have a crucial problem that, in produced hepatocytes, the cytochrome P450 enzymatic activity is already detectable even in the absence of any drugs and drug-induced up-regulation in the cytochrome P450 enzymatic activity is not detected (Non-Patent Literatures 3 to 8).

Furthermore, conventional technologies leave severe problems unsolved: 1) culture systems contain fetal bovine serum, 2) culture systems also contain feeder cells derived from mice, and 3) human pluripotent stem cell lines highly differ from each other in the differentiation propensity (Non-Patent Literature 9).

If an evaluation system contains fetal bovine serum, the drug added to the evaluation system attaches to the serum components, resulting in impossibility of correctly evaluating the original metabolism/toxicity of the drug. Similarly, an evaluation system containing mouse-derived cells has a risk of the system of measuring drug metabolism/toxicity being disturbed. In addition, since the widely varying efficiency of producing hepatocytes between cell lines makes the system of measuring drug metabolism/toxicity unstable, verification of difference in individuals from which cell lines are derived becomes impossible.

Accordingly, in order to appropriately and effectively perform drug metabolism/toxicity testing, it is urgently necessary to develop functional hepatocytes that allow detection of a change in the activity of cytochrome P450 enzyme in response to the addition of a drug under serum-free and feeder-free conditions, from human pluripotent stem cells by a stable culture technology devoid of producing line difference.

### Citation List

### Patent Literature

[Patent Literature 1] Japanese Patent Laid-Open No. 2010-75199, "Culture system for rapid expansion of human embryonic stem cell"
[Patent Literature 2] Japanese Patent Laid-Open No. 2008-212150, "Human embryonic stem cell-originated pancreas islet cell"
[Patent Literature 3] National Publication of International Patent Application No. 2010-529851, "Multipotent/pluripotent cells and method"

### Non-Patent Literature

[Non-Patent Literature 1] Nicholls H, Drug Discovery Today, vol. 8, p. 1055
[Non-Patent Literature 2] Gripon P, et al., Proceedings of the National Academy of Sciences of the United States of America, vol. 99, pp. 15655-15660
[Non-Patent Literature 3] Inamura M, et al., Molecular Therapy, (first edition), 2010
[Non-Patent Literature 4] Liu H, et al., Hepatology, vol. 51, pp. 1810-1819
[Non-Patent Literature 5] Sullivan GJ, et al., Hepatology, vol. 51, pp. 329-335
[Non-Patent Literature 6] Touboul T, et al., Hepatology, vol. 51, pp. 1754-1765
[Non-Patent Literature 7] Duan Y, et al., Stem Cells, vol. 28, pp. 674-686
[Non-Patent Literature 8] Hay DC, et al., Proceedings of the National Academy of Sciences of the United States of America, vol. 105, pp. 12301-12306
[Non-Patent Literature 9] Osafune K, et al., Nature Biotechnology, vol. 26, pp. 313-315
[Non-Patent Literature 10] Experimental Medicine, Vol. 26, No. 5 (Supplement), pp. 35-40, 2008
[Non-Patent Literature 11] Mitaka T, et al., Biochemical and Biophysical Research Communications, vol. 214, pp. 310-317, 1995
[Non-Patent Literature 12] Chen Q, et al., Nature Protocol, vol. 2, pp. 1197-1205, 2007
[Non-Patent Literature 13] Zhao D, et al., PLoS One, vol. 4, e6468, 2009

### Summary of Invention

### Technical Problem

It is a major object of the present invention to provide a technology for stably producing high-quality functional hepatocytes from pluripotent stem cells. In particular, it is an object of the present invention to produce high-quality functional hepatocytes, from human pluripotent stem cells, to be used in testing of metabolism and toxicity of a drug.

It is also an object of the present invention to provide a technology for stably producing functional hepatocytes that allow detection of a change in the activity of cytochrome P450 enzyme in response to the addition of a drug, from human pluripotent stem cells, under serum-free and feeder-free conditions without causing differences among human pluripotent stem cell lines.

It is also an object of the present invention, in also the viewpoint of personalized medicine, to provide an in vitro system for evaluating individual difference in hepatotoxicity of a drug by providing a technology for stably producing functional hepatocytes that allow detection of a change in the activity of cytochrome P450 enzyme in response to the addition of a drug, under serum-free and feeder-free conditions without line difference, from human iPS cells established from somatic cells from an appropriate individual without causing genomic insertion of a foreign gene by means of a Sendai virus vector.

### Solution to Problem

The present inventors have found that preparation of human pluripotent stem cells seeded in advance at an appropriate density is a key issue for culture of inducing differentiation of human pluripotent stem cells into hepatocytes.
Specifically, the inventors have found that a density such that agglomerates of human pluripotent stem cells are just in contact with one another is optimum. This enables a stable implementation of differentiation of pluripotent stem cells into hepatocytes with high efficiencies and without line difference, which was conventionally very difficult to prepare and caused a large variation among cell lines.

The present inventors have also found that addition of serum or serum replacement, which has been conventionally performed, significantly reduces the efficiency of differentiation induction in culture of inducing differentiation of human pluripotent stem cells into hepatocytes.
The lack of serum and serum replacement, however, significantly reduces the viability of human pluripotent stem cells. It was therefore found that the culture of inducing differentiation of human pluripotent stem cells into hepatocytes involves a dilemma that in both the presence and absence of serum or serum replacement, the efficiency of differentiation induction is significantly lowered and become unstable.
In order to solve this problem, the present inventors have found that addition of human recombinant albumin to a culture system in the early stage of differentiation induction is highly effective for increasing the viability without reducing the efficiency of inducing differentiation of human pluripotent stem cells into hepatocytes.

The inventors also have found that addition of sonic hedgehog (SHH) protein, which is known as a promoter of differentiation into liver from a common ancestor for liver and pancreas during murine embryogenesis, to a culture system of inducing differentiation of human pluripotent stem cells into hepatocytes is effective for increasing efficiency and stability of the differentiation induction.

The inventors have also found that separation taking advantage of difference in sedimentation velocity is effective as a method for removing feeder cells (mouse fetal fibroblasts) from human pluripotent stem cells, in the undifferentiated state prior to differentiation induction with high efficiency and without causing damage in the cells. As a result, differentiation of human pluripotent stem cells into hepatocytes can be efficiently and rapidly induced under feeder-free conditions.

Based on these findings, the present invention provides the following constituents. That is, the method of producing pluripotent stem cell-derived highly functional hepatocytes of the present invention is a method of producing highly functional hepatocytes using pluripotent stem cells, wherein pluripotent stem cell-derived primitive endoderms are prepared from pluripotent stem cells by a method including the steps (A) and (B); liver progenitor cells are prepared from the pluripotent stem cell-derived primitive endoderms by a method including the step (C); and highly functional hepatocytes are prepared from the liver progenitor cells by a method including the step (D):
(A) culture under serum-free and feeder-free conditions;
(B) culture in the presence of albumin and at least one cytokine;
(C) culture in the presence of SHH or an SHH agonist and at least one cytokine; and
(D) culture to promote maturity in the presence of at least one cytokine.

The method of producing pluripotent stem cell-derived primitive endoderms of the present invention is a method of producing pluripotent stem cell-derived primitive endoderms using pluripotent stem cells, wherein pluripotent stem cell-derived primitive endoderms are prepared from pluripotent stem cells by a method including the steps (A) and (B):
(A) culture under serum-free and feeder-free conditions; and
(B) culture in the presence of albumin and at least one cytokine.

Here, in the step (A), the pluripotent stem cells may be seeded at a density such that agglomerates of the cells are in contact with one another.

In the step (A), the feeder cells may be removed by separation utilizing the difference in sedimentation velocity.

In the step (B), albumin may be human recombinant albumin. '

The method of producing liver progenitor cells of the present invention is a method of producing liver progenitor cells using primitive endoderms, wherein liver progenitor cells are prepared from primitive endoderms by a method including the step (C):
(C) culture in the presence of SHH or an SHH agonist and at least one cytokine.

Here, in the step (C), the primitive endoderms may be pluripotent stem cell-derived primitive endoderms.

The method of producing highly functional hepatocytes of the present invention is a method of producing highly functional hepatocytes using liver progenitor cells, wherein highly functional hepatocytes are produced from liver progenitor cells by a method including the step (D):
(D) a step of culture to promote maturity in the presence of at least one cytokine.

In the above, the iPS cells may be those established with a Sendai virus vector.

The pluripotent stem cells may be human pluripotent stem cells.

The pluripotent stem cell-derived highly functional hepatocytes of the present invention are produced from pluripotent stem cells, wherein pluripotent stem cell-derived primitive endoderms are prepared from pluripotent stem cells by a method including the steps (A) and (B); liver progenitor cells are prepared from the pluripotent stem cell-derived primitive endoderms by a method including the step (C); and the highly functional hepatocytes are produced from the liver progenitor cells by a method including the step (D):
(A) culture under serum-free and feeder-free conditions;
(B) culture in the presence of albumin and at least one cytokine;
(C) culture in the presence of SHH or an SHH agonist and at least one cytokine; and
(D) culture to promote maturity in the presence of at least one cytokine.

The pluripotent stem cell-derived highly functional hepatocyte of the present invention is produced from a pluripotent stem cell, shows a cytochrome P450 enzymatic activity in the presence of dexamethasone at a level twice or more that of a HepaRG cell, has ICG uptake and release activities, is PAS staining positive, expresses A1AT, and can also differentiate into a bile duct epithelial cell.

The pluripotent stem cell-derived primitive endoderms of the present invention are produced from pluripotent stem cells, wherein the pluripotent stem cell-derived primitive endoderms are prepared from pluripotent stem cells by a method including the steps (A) and (B):
(A) culture under serum-free and feeder-free conditions; and
(B) culture in the presence of albumin and at least one cytokine.

The liver progenitor cells of the present invention are produced using primitive endoderms, wherein the liver progenitor cells are prepared from primitive endoderms by a method including the step (C):
(C) culture in the presence of SHH or an SHH agonist and at least one cytokine.

The highly functional hepatocytes of the present invention are produced using liver progenitor cells, wherein the highly functional hepatocytes are produced from liver progenitor cells by a method including the step (D):
(D) culture to promote maturity in the presence of at least one cytokine.

In the above, the pluripotent stem cells may be ES cells or iPS cells.

The pluripotent stem cells may be human pluripotent stem cells.

The method of testing drug metabolism of the present invention is characterized in that the method quantitatively evaluates the metabolism of a drug utilizing highly functional hepatocytes produced from pluripotent stem cells described in any of the above.

The method of testing drug toxicity of the present invention is characterized in that the method quantitatively evaluates the cell death due to a drug utilizing highly functional hepatocytes produced from pluripotent stem cells described in any of the above.

### Advantageous Effects of Invention

The present invention can provide a technology of stably producing high-quality functional hepatocytes from pluripotent stem cells.

The present invention enables stable production of functional hepatocytes that allow detection of a change in the activity of cytochrome P450 enzyme in response to the addition of a drug under serum-free and feeder-free conditions without line difference among human pluripotent stem cells, from human pluripotent stem cells.

### Brief Description of Drawings

[Fig. 1] Fig. 1 includes microscopic photographs showing changes in the morphology of a SeV-iPS cell.
[Fig. 2] Fig. 2 includes graphs showing fluorescence activated cell scanning (FACS) results showing expression of each of the undifferentiation markers, SSEA4 and OCT4, in SeV-iPS cells.
[Fig. 3] Fig. 3 includes photographs of immunostaining showing expression of each of the undifferentiation markers, SSEA4, OCT3/4, and Nanog, in SeV-iPS cells.
[Fig. 4A] Fig. 4A includes photographs of electrophoresis showing removal of SeV vector-derived foreign genes in SeV-iPS cells.
[Fig. 4B] Fig. 4B includes microscopic photographs showing removal of a SeV vector-derived foreign gene in a SeV-iPS cell.
[Fig. 5] Fig. 5 is a phase contrast microscopic photograph of hepatocytes derived from human iPS cells (cell line #40).
[Fig. 6] Fig. 6 is a table showing primers for RT-PCR for detecting hepatocyte markers.
[Fig. 7] Fig. 7 is a photograph of electrophoresis of RT-PCR products from hepatocytes differentiated from pluripotent stem cells.
[Fig. 8] Fig. 8 includes graphs of quantitative RT-PCR products from hepatocytes differentiated from pluripotent stem cells.
[Fig. 9] Fig. 9 includes photographs of immunostained hepatocytes differentiated from pluripotent stem cells.
[Fig. 10] Fig. 10 includes photographs of Western blotting of hepatocytes differentiated from pluripotent stem cells.
[Fig. 11] Fig. 11 includes microscopic photographs by PAS staining of hepatocytes differentiated from pluripotent stem cells.
[Fig. 12] Fig. 12 includes microscopic photographs showing the ICG uptake and release by hepatocytes differentiated from pluripotent stem cells.
[Fig. 13] Fig. 13 is a graph showing cytochrome P450 activity in hepatocytes differentiated from pluripotent stem cells.
[Fig. 14A] Fig. 14A is a microscopic photograph of a hepatocyte differentiated from a pluripotent stem cell.
[Fig. 14B] Fig. 14B is a microscopic photograph of a hepatocyte differentiated from a pluripotent stem cell.
[Fig. 14C] Fig. 14C is a microscopic photograph of a hepatocyte differentiated from a pluripotent stem cell.

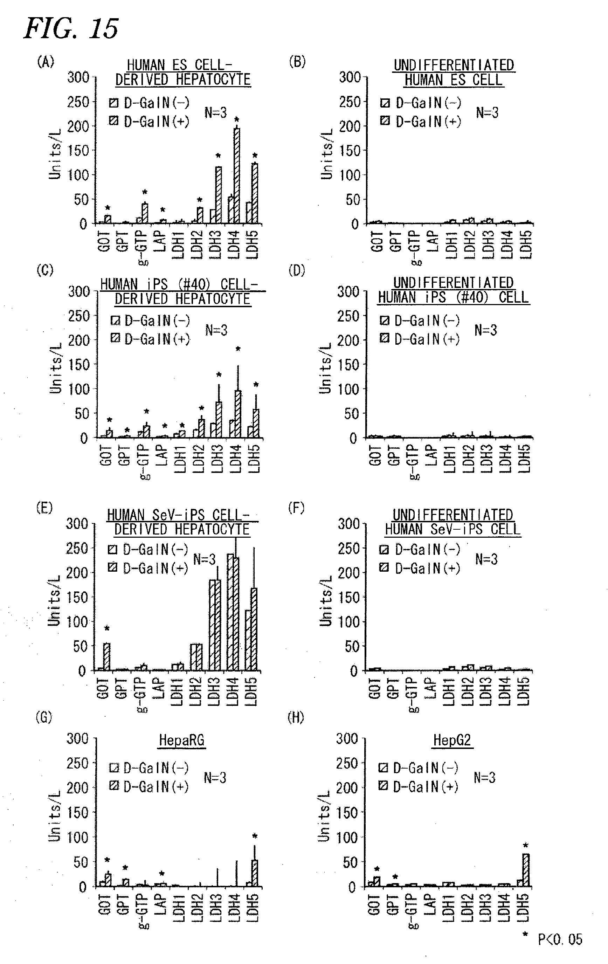

[Fig. 15] Fig. 15 includes graphs (A) and (B) showing the results of drug toxicity testing using human embryonic stem cell-derived hepatocytes and undifferentiated human embryonic stem cells, respectively; graphs (C) and (D) showing the results of drug toxicity testing using human iPS cell-derived hepatocytes and undifferentiated human iPS cells, respectively; graphs (E) and (F) showing the results of drug toxicity testing using human SeV-iPS cell-derived hepatocytes and undifferentiated human SeV-iPS cells, respectively; and graphs (G) and (H) showing the results of drug toxicity testing using HepaRG cells and HepG2 cells, respectively.
[Fig. 16] Fig. 16 is a graph showing the specificity of toxicity to hepatocytes of D-GalN.
[Fig. 17] Fig. 17 includes (A) microscopic photographs showing the morphology of a human iPS cell-derived hepatocyte (#40) and (B) microscopic photographs showing the morphology of a human embryonic stem cell-derived hepatocyte (KhES-1).

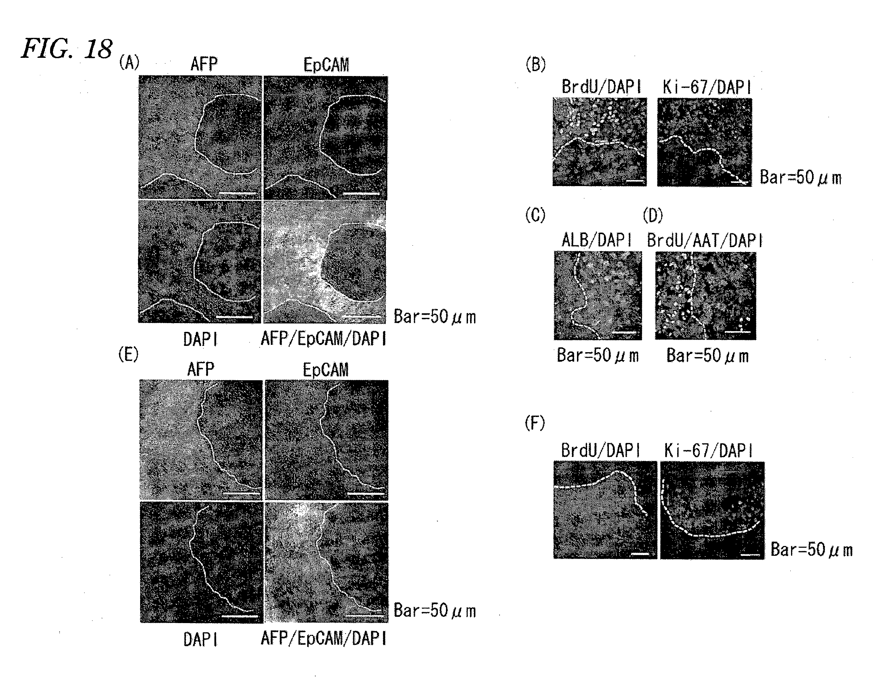

[Fig. 18] Fig. 18 includes photographs (A) showing immunostaining of EpCAM and α-FP in human induced pluripotent stem cell-derived hepatocytes; photographs (B) showing immunostaining of BrdU and Ki67 in human induced pluripotent stem cell-derived hepatocytes; a photograph (C) showing immunostaining of Alb in human induced pluripotent stem cell-derived hepatocytes; a photograph (D) showing immunostaining of AAT in human induced pluripotent stem cell-derived hepatocytes; photographs (E) showing immunostaining of EpCAM and α-FP in human induced pluripotent stem cells; and photographs (F) showing immunostaining of BrdU and Ki67 in human induced pluripotent stem cells.
[Fig. 19] Fig. 19 includes microscopic photographs (A), (B), (C), and (D) of liver stem/progenitor cell populations in hepatocytes induced from human embryonic stem cells and human induced pluripotent stem cells, after subculture; and photographs (E) of electrophoresis of RT-PCR products showing expressions of α-FP, TDO2, and AAT.

### Description of Embodiments

Embodiments of the present invention will now be described based on examples shown in figures. Note that embodiments are not limited to the following exemplary examples and can be appropriately designed or modified using conventionally known technologies, such as the above-mentioned documents, within the scope of the gist of the present invention.

The highly functional hepatocytes of the present invention are produced from pluripotent stem cells, where pluripotent stem cell-derived primitive endoderms are prepared from pluripotent stem cells by a method including the steps (A) and (B); liver progenitor cells are prepared from the pluripotent stem cell-derived primitive endoderms by a method including the step (C); and the highly functional hepatocytes are produced from the liver progenitor cells by a method including the step (D):
(A) culture under serum-free and feeder-free conditions;
(B) culture in the presence of albumin and at least one cytokine;
(C) culture in the presence of SHH or an SHH agonist and at least one cytokine; and
(D) culture to promote maturity in the presence of at least one cytokine.

Pluripotent stem cell-derived highly functional hepatocytes are typically prepared from pluripotent stem cells by separating the pluripotent stem cells from feeder cells such as mouse fetal fibroblasts; seeding the pluripotent stem cells on a culture plate coated with, for example, Matrigel (registered trademark) (manufactured by Becton, Dickinson and Company) at a density such that agglomerates of the cells are just in contact with one another; culturing the cells for about three days in a undifferentiation-maintaining medium; and then culturing the cells in a medium for mesendoderm differentiation, a medium for embryonic endoderm differentiation, a medium for inducing differentiation into liver progenitor cells, and a medium for promoting maturity of hepatocytes, sequentially.
The term for producing the pluripotent stem cell-derived highly functional hepatocytes from pluripotent stem cells is about 20 to 30 days.

The medium for differentiation and the medium for promoting maturity are each a generic basal medium or serum-free culture medium containing at least one cytokine at a concentration of about 10 to 200 ng/mL or at least one hormone at a concentration of about 0.1 µM. These media may optionally contain appropriate other additives as far as it does not adversely affect the maintenance and differentiation of cells. Examples of the generic basal medium include DMEM, DMEM/F12, RPMI, and IMDM. Examples of the serum-free culture medium include S-Clone SF-B (manufactured by Sanko Junyaku Co., Ltd.), S-Clone SF-03 (manufactured by Sanko Junyaku Co., Ltd.), ASF-104 (manufactured by Ajinomoto Co., Inc.), ASF-104N (manufactured by Ajinomoto Co., Inc.), X-VIVO 10 (manufactured by Lonza Inc.), and X-VIVO 15 (manufactured by Lonza Inc.), and the like.

The medium for differentiation and the medium for promoting maturity may contain any fundamental culture ingredients that are suitable for inducing differentiation from human pluripotent stem cells into hepatocytes, and examples of the ingredients include Roswell Park Memorial Institute (RPMI) 1640, and the like.
The culturing conditions in differentiation induction can be appropriately determined depending on the type of the human pluripotent stem cells to be used, and examples of the conditions include conditions of a temperature of 37°C and 5% by volume of carbon dioxide gas culture apparatus (5% CO₂ incubator).

The present invention will now be described in detail.
The pluripotent stem cell-derived embryonic endoderms used in the present invention are embryonic endoderms derived from pluripotent stem cells. The embryonic endoderm is an endodermal component derived from a mesendoderm formed after gastrulation in early development, contributes to the formation of fetal digestive tract tissue, and expresses a transcription factor such as SOX17 or FOXA2. The embryonic endoderm can be prepared by a method including the steps (A) and (B):
(A) culture under serum-free and feeder-free conditions; and
(B) culture in the presence of albumin and at least one cytokine.

Each step will now be described.

### (A) A step of culture under serum-free and feeder-free conditions

A pluripotent stem cell refers to a stem cell having pluripotency, and examples thereof include ES cells, iPS cells, testicular stem cells, adult stem cells, and Muse cells.
The pluripotent stem cells as a starting material may be human pluripotent stem cells. In such a case, the pluripotent stem cells are derived from a human and form a cell population preserving pluripotent differentiation ability. Examples of the pluripotent stem cells include human ES cells, human iPS cells, human testicular stem cells, human adult stem cells, and human Muse cells.

The pluripotent stem cells as a starting material can be acquired or established by known methods. For example, human ES cells can be obtained from domestic establishment institutions (Kyoto University, National Center for Child Health and Development) with permission of the Ministry of Education, Culture, Sports, Science and Technology or can be obtained or purchased from foreign institutions (such as private companies and universities). Human iPS cells can be established by, preferably, a method using a Sendai virus (SeV) vector and are established by, for example, culturing commercially available human fibroblasts in a medium containing a Sendai virus vector carrying a reprogram factor-expressing unit. Examples of the Sendai virus vector carrying a reprogram factor-expressing unit include CytoTune-iPS (manufactured by DNAVEC Corporation). Human iPS cells produced using a retrovirus vector can be purchased from RIKEN BioResource Center or can be obtained from Kyoto University or National Center for Child Health and Development.

The undifferentiation-maintaining medium used in the step (A) may be a generic medium for maintaining undifferentiation and is more preferably an undifferentiation-maintaining medium not containing any cytokine such as fibroblast growth factor 2 (FGF 2).
The term "culture under serum-free and feeder-free conditions" means culture in a medium not containing any animal serum and human serum under conditions in the absence of cell species (e.g., mouse fetal fibroblasts) other than the pluripotent stem cells. On this occasion, the most preferable culture density of the pluripotent stem cells is a density such that agglomerates of the pluripotent stem cells are just in contact with one another.
A density lower than this level causes massive cell death in the early stage of differentiation induction or causes differentiation into non-endoderm lineage cells with many dendrites even if the cells survive. In contrast, a density higher than the level leads to massive cell death from the region where a three-dimensional construction specific to endoderm-derived tissue would otherwise be emerging at later phases of differentiation.

### (B) A step of culture in the presence of albumin and at least one cytokine

The albumin is preferably human recombinant albumin, and the amount of the albumin is about 0.1 to 1%, preferably about 0.3 %.
Examples of the cytokine used in the step (B) include Wnt3A and Activin A. The amount of the cytokine is, for example, in the case of Wnt3A, about 10 to 50 ng/mL, preferably about 25 ng/mL, and in the case of Activin A, about 10 to 100 ng/ml, preferably about 100 ng/mL.
The pluripotent stem cell-derived mesendoderms can be prepared by culturing the undifferentiated pluripotent stem cells prepared in the step (A) in a medium containing human recombinant albumin, Wnt3A, and Activin A at 37°C in a 5% CO₂ incubator for about one day. The cells are further cultured in a medium containing Activin A and serum replacement at 37°C in a 5% CO₂ incubator for about one day to give pluripotent stem cell-derived embryonic endoderms. Examples of the serum replacement include KnockOut (registered trademark) Serum Replacement (manufactured by Life Technologies, Inc.).

Production of pluripotent stem cell-derived embryonic endoderms can be confirmed by verification of induced expression of a transcription factor, which is crucial for embryonic endoderm formation, such as SOX17 and FOXA2, by RT-PCR.

The liver progenitor cell used in the present invention is a cell in which commitment from a primitive gut to a hepatocyte occurred and can be prepared by a method including the step (C).

### (C) A step of culture in the presence of SHH or an SHH agonist and at least one cytokine

The SHH and the SHH agonist are each a recombinant peptide or agent that activates a sonic hedgehog pathway. Examples of the SHH include N-terminal peptide fragment (Cys24-Gly197) of human sonic hedgehog protein. Examples of the SHH agonist include a chlorobenzothiophene-containing hedgehog agonist (SAG). The concentration of the sonic hedgehog N-terminal peptide fragment is, for example, 100 to 400 ng/mL, preferably about 200 ng/mL. The concentration of SAG is, for example, 1 nM to 500 nM, preferably about 100 nM.

Examples of the cytokine used in the step (C) include fibroblast growth factor 2 (FGF 2), bone morphogenetic factor 4 (BMP 4), hepatocyte growth factor (HGF), and the like. The amount of the cytokine is, for example, in the case of FGF 2, 5 to 50 ng/mL, preferably about 10 ng/mL, in the case of BMP 4, 5 to 50 ng/mL, preferably about 20 ng/mL, and in the case of HGF, 5 to 50 ng/mL, preferably about 20 ng/mL. Specifically, pluripotent stem cell-derived liver progenitor cells can be prepared by culturing the pluripotent stem cell-derived embryonic endoderms prepared in the step (B) in, for example, a culture solution of a basal medium, such as RPMI 1640 containing 2% serum replacement, containing FGF 2, BMP 4, and SHH for about 5 days and then culturing in a culture solution of a basal medium, such as RPMI 1640 containing 2% serum replacement, containing FGF 2, BMP 4, and HGF for about 4 days. Here, the former culture is performed using a culture solution containing SHH, and the latter culture is performed using a culture solution not containing SHH, but the latter culture may be performed in a culture solution containing SHH. In addition, the medium is preferably replaced by fresh one at least once during each culture period.

Production of pluripotent stem cell-derived liver progenitor cells can be confirmed by verification of induced expression of a transcription factor, which is crucial for hepatic primordium formation, such as HNF4a, or a marker gene of a hepatic primordium such as α-fetoprotein by, for example, RT-PCR or Western blotting.

The pluripotent stem cell-derived highly functional hepatocytes of the present invention can be prepared by a method including the step (D).

### (D) A step of culture to promote maturity in the presence of at least one cytokine

Examples of the cytokine and the hormone used here include oncostatin M (OSM) and dexamethasone. The amount thereof is, for example, in the case of OSM, 10 to 50 ng/mL and preferably about 25 ng/mL and in the case of dexamethasone, 0.05 to 0.5 µM and preferably about 0.1 µM.
The pluripotent stem cell-derived highly functional hepatocytes can be prepared by culturing the pluripotent stem cell-derived liver progenitor cells prepared in the step (C) in, for example, a culture solution of a basal medium, such as RPMI 1640 containing 2% serum replacement, containing OSM and dexamethasone for about 2 weeks. The medium is preferably replaced by fresh one at a frequency of once every 3 days during the culture period.

Thus-prepared pluripotent stem cell-derived highly functional hepatocyte shows, when a high concentration (about 10 µM) of dexamethasone is present, the activity level of a cytochrome P450 enzyme which is twice or more that of the HepaRG cell. The pluripotent stem cell-derived highly functional hepatocyte has indocyanine green (ICG) uptake and release activities, is Periodic Acid Schiff (PAS) staining positive, expresses α1-antitrypsin (A1AT), and can also differentiate into a bile duct epithelial cell.

The activity level of a cytochrome P450 enzyme, in the presence of a high concentration of dexamethasone, of the pluripotent stem cell-derived highly functional hepatocyte being twice or more that of the HepaRG cell can be verified by a test using, for example, a generic kit for detecting the activity of a cytochrome P450 enzyme. For example, p450-Glo^{™} CYP Assay kit of Promega Corporation measures cytochrome P450 enzymatic activity as a quantity of light with a luminometer. Accordingly, the ratio of the activity level of cytochrome P450 enzyme of a pluripotent stem cell-derived highly functional hepatocyte to that of a HepaRG cell can be calculated as a ratio of quantities of light.

The ICG-uptaking activity can be confirmed by adding, for example, 1 mg/mL of ICG to pluripotent stem cell-derived highly functional hepatocytes, culturing the hepatocytes at 37°C in a 5% CO₂ incubator for 30 minutes, then washing the hepatocytes with a colorless buffer not containing ICG, such as PBS, and then observing under an optical microscope, that the hepatocytes have been stained into green with the ICG dye.

The ICG-releasing activity can be confirmed by culturing the above ICG-stained cells in a medium for promoting maturity of hepatocytes, for example, at 37°C in a 5% CO₂ incubator for 6 hours and then observing under an optical microscope, that the cells have become colorless by releasing the ICG dye.

The PAS staining positivity can be confirmed by fixing the pluripotent stem cell-derived highly functional hepatocytes with about 10% formalin, performing PAS staining by a generic method, detecting reddish purple granules in the cytoplasm by an observation under an optical microscope, thereby confirming the presence of glycogen granules in the pluripotent stem cell-derived highly functional hepatocytes.

Expression of A1AT can be confirmed by being positive in all of three tests: RT-PCR of RNA prepared from cells using a primer specific to A1AT, Western blotting of a lysate of cells using an antibody specific to A1AT, and immunostaining of fixed cells using an antibody specific to A1AT.

Ability to differentiate into bile duct epithelial cells can be determined by the presence of a cell population positive for an oval cell marker, EpCAM, and a cell population positive for a bile duct epithelial cell marker (such as γ-GTP, ALP-1, or LAP), together with the highly functional hepatocytes, in the cells produced in the step (D).

The formation of a bile duct structure by the bile duct epithelial cells can be confirmed by detecting the connection of a tubular structure composed of ciliated epithelial cells to a Hering duct by an observation under an electron microscope.

The thus-prepared hepatocytes are pluripotent stem cell-derived highly functional hepatocytes and have excellent properties such that they are free from contamination with heterologous animal cells and infection with heterologous animal-derived viruses. The thus-prepared pluripotent stem cell-derived highly functional hepatocytes are functional hepatocytes characterized with features such as; the activity level of a cytochrome P450 enzymatic activity in the presence of a high concentration of dexamethasone is twice or more that of HepaRG cells, and the increase in the activity level of the cytochrome P450 enzyme in response to the addition of a high concentration of dexamethasone is detected by a statistically significant difference; and the highly functional hepatocytes have ICG-uptaking and releasing activities, are PAS staining positive, express A1AT, and can also differentiate into bile duct epithelial cells.
A major feature of the pluripotent stem cell-derived highly functional hepatocytes produced by the present invention is that these hepatocytes are guaranteed stably have high quality regardless of lines of the pluripotent stem cells. In this point, the problem of inter-lot differences in liver biopsy materials sampled from donors and their culture products (primary cultured hepatocytes) is completely overcome. In addition, in liver biopsy materials and primary cultured hepatocytes, their functions are considerably deteriorated by long-term culture, and it is therefore difficult to supply them corresponding to a demand. However, the pluripotent stem cell-derived highly functional hepatocytes can be easily supplied corresponding to a demand.

The prepared highly functional hepatocytes derived from pluripotent stem cells can be used for tests of metabolism and toxicity of drugs.
For example, in a case of use for testing metabolism, a target drug is added to the pluripotent stem cell-derived highly functional hepatocytes, followed by culturing, for example, at 37°C in a 5% CO₂ incubator for about 16 hours and detecting the activity of a cytochrome P450 enzyme with a generic kit. For example, in a case of using a p450-Glo^{™} CYP Assay kit of Promega Corporation, the assay can be performed using 50µL of a culture supernatant, and it is therefore possible to evaluate the cytochrome P450 enzymatic activities over time after a differentiation induction of pluripotent stem cells into hepatocytes.

In a case of using the hepatocytes for testing toxicity, a target drug is added to the pluripotent stem cell-derived highly functional hepatocytes, followed by culturing, for example, at 37°C in a 5% CO₂ incubator, and the cytotoxicity of the drug can be evaluated over time using a generic technology for measuring cell death. Examples of the generic technology for measuring cell death include detection of chromosomal DNA fragments by electrophoresis, detection of cells having reduced chromosomal DNA by flow cytometry, detection of apoptosis cells with Annexin V, and detection of dead cells with a dead cell-staining agent such as propidium iodide (PI).

Furthermore, the toxicity to hepatocytes can be distinguished from the toxicity to bile duct epithelial cells by adding a target drug to the pluripotent stem cell-derived highly functional hepatocytes, culturing the hepatocytes, for example, at 37°C in a 5% CO₂ incubator, collecting the culture supernatants over time, and measuring enzymes characteristic to hepatocytes (such as glutamic oxaloacetic transaminase (GOT) and glutamic pyruvic transaminase (GPT)) and enzymes characteristic to bile duct epithelial cells (such as γ-glutamyl transpeptidase (γ-GTP), leucine aminopeptidase (LAP), and alkaline phosphatase 1 (ALP-1)) by generic methods.

Testing the toxicity of a drug using the pluripotent stem cell-derived hepatocytes will be described in more detail by the following preferable method.
A drug in various concentrations (for example, 5 to 10 mM of acetaminophene) is added to culture supernatants of pluripotent stem cell-derived hepatocytes. Subsequently, the hepatocytes are cultured, for example, at 37°C in a 5% CO₂ incubator. The toxicity of the drug can be quantitatively evaluated by measuring the cell viability and the cell death over time.
Examples of the quantitative measurement of cell viability include a generic method of measuring mitochondrial respiration capacity using a tetrazolium salt as a substrate (such as MTT assay and WST assay).

Examples of the quantitative measurement of cell death include 1) generic technologies for detecting apoptosis (e.g., detection of apoptosis cells by flow cytometry using Annexin V as a probe, measurement of cellular DNA content with PI (i.e., measurement of sub G1 fraction), quantitative measurement of chromosomal low molecular weight DNA by agarose electrophoresis, and TUNEL assay), 2) generic technologies for detecting cell death (measurement of PI-positive cells by flow cytometry and measurement of the amount of chromosomal DNA degradation by comet assay), and 3) generic methods of measuring deviation enzymes (e.g., glutamic oxaloacetic transaminase (GOT), glutamic pyruvic transaminase (GPT), γ-glutamyl transpeptidase (γ-GTP), leucine aminopeptidase (LAP), or alkaline phosphatase (ALP)) in culture supernatants.
Among these methods, the measurement of deviation enzymes in a culture supernatant is particularly excellent in that it can measure the toxicity to hepatocytes and the toxicity to duct cells separately. That is, the toxicity to hepatocytes and the toxicity to duct cells can be separately evaluated by measuring hepatocyte-selective enzymes (GOT or GPT) characteristic to hepatocytes and bile duct epithelial cell-selective enzymes (γ-GTP, LAP, or ALP-1), respectively.

### Examples

### [Example 1] Induction of human induced pluripotent stem cell using Sendai virus vector

First, 5.0×10⁵ cells of human newborn foreskin fibroblasts (BJ) (ATCC (http://www.atcc.org), CRL-2522) were cultured in a 10% FBS-containing DMEM (Life Technologies, Inc., Grand Island, NY, USA) at 37°C in a 5% CO₂ incubator. Subsequently, the cultured cells were infected with the following vectors (a) to (d) in a concentration of MOI=3:
(a) SeV18+ OCT3/4/TSΔF vector
(b) SeV18+ SOX2/TSΔF vector
(c) SeV18+ KLF4/TSΔF vector
(d) SeVHNL c-MYC/TS15ΔF vector

On the following day of the infection with the vectors, the medium was replaced by a 10% FBS-containing DMEM, followed by culture at 37°C in a 5% CO₂ incubator for 6 days. Subsequently, cells transfected with the vectors were detached with Accutase and were cultured on mouse fetal fibroblasts (MEFs), which had been prepared on a gelatin-coated 10-cm culture plate, at 5.0×10⁴ to 5.0×10⁵ per 10-cm culture plate. On the following day, the 10% FBS-containing DMEM was replaced by a medium for primate ES cells (ReproCELL Inc., Tokyo, Japan) (containing 5 ng/mL of FGF 2), followed by culture in a 3% CO₂ incubator. The medium was replaced every day.

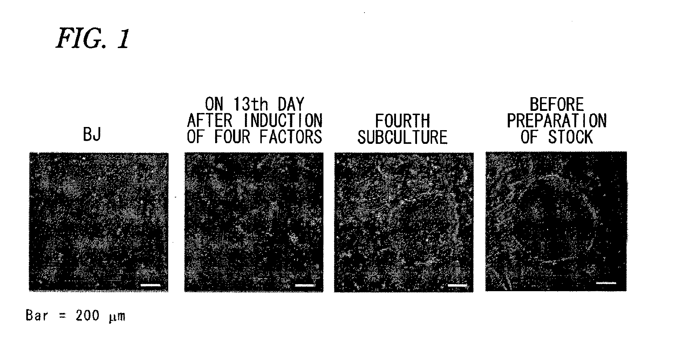

Fig. 1 includes microscopic photographs showing morphological changes of a BJ cell-derived human induced pluripotent stem cells (SeV-iPS cells).
Colonies appeared after several days of the culture, and human embryonic stem cell-like colonies appeared by culture for about 20 days. As shown in photographs of Fig. 1, flat colonies, which were obviously different from BJ cells before the induction, but similar to those observed in human embryonic stem cells, were detected. The human embryonic stem cell-like colonies had the same appearance as those conventionally reported (Non-Patent Literature 10). These colonies were isolated with a micropipette and were then cultured on a fresh MEF layer. The cells could stably be passaged and expanded by subculture through a dissociation procedure using a human pluripotent stem cell dissociation solution (0.25% trypsin (manufactured by Life Technologies, Inc.), 1 mg/mL collagenase IV (manufactured by Wako Pure Chemical Industries, Ltd.), 20% KnockOut (registered trademark) Serum Replacement (manufactured by Life Technologies, Inc.), and 1 mM CaCl₂).

In order to show whether or not the cells prepared by the experiment above expressed markers characteristic to pluripotent stem cells or not, the following experiment was further performed.

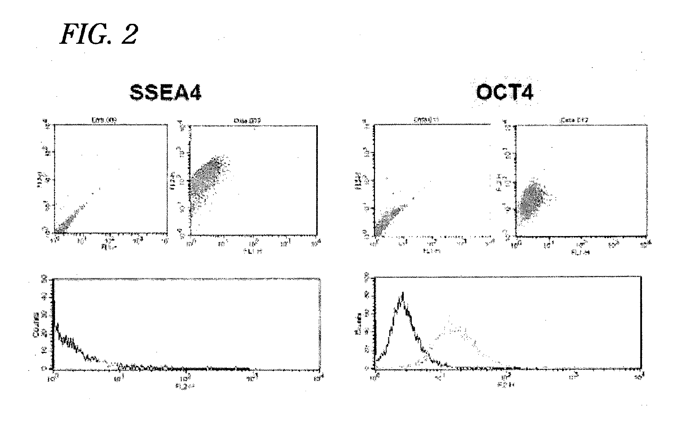

[Example 2] Confirmation that human induced pluripotent stem cell established by using Sendai virus vectors were maintained in an undifferentiated state.
Expressions of SSEA4 and OCT3/4, which are markers of undifferentiated human pluripotent stem cells, were investigated with a flow cytometer (FACSCalibur (registered trademark)) (BD Biosciences).
Specifically, in the case of SSEA4, the SeV-iPS cells prepared in Example 1 were collected by a treatment with a pluripotent stem cell dissociation solution (0.25% trypsin (Life Technologies, Inc.), 1 mg/mL collagenase IV (manufactured by Wako Pure Chemical Industries, Ltd.), 20% KnockOut (registered trademark) Serum Replacement (manufactured by Life Technologies, Inc.), and 1 mM CaCl₂) and were dispersed by a treatment with trypsin/EDTA solution (Sigma Chemical Co., St. Louis, MO, USA) and then floated in a FACS buffer (×1 PBS, 0.05% NaN₃, 5% FBS). To the suspension, 2% mouse BD Fc Block (BD Biosciences) is added and then anti-human SSEA4 phycoerythrin conjugated mouse IgG (R&D, Minneapolis, MN, USA) diluted to 1/10 were added. The mixture was left to stand on ice for 60 minutes. After washing with a FACS buffer, the expression of SSEA4 was analyzed with a flow cytometer.
In the case of OCT3/4 expression, the collected SeV-iPS cells were subjected to cell fixation and cell membrane permeabilization with FIX & PERM CELL PERMEABILIZATION KIT (Caltag Laboratories, An-Der-Grub, Austria), and 2% mouse BD Fc Block (BD Biosciences) and anti-human OCT3/4 phycoerythrin conjugated rat IgG (R&D, Minneapolis, MN) diluted to 1/10 were added thereto. The mixture was left to stand on ice for 60 minutes. After washing with a FACS buffer, the expression of OCT3/4 was analyzed with a flow cytometer.
Consequently, as shown in the results of FACS shown in Fig. 2, it was confirmed that the SeV-iPS cells prepared in Example 1 highly express the undifferentiation markers, SSEA4 and OCT4. The results regarding OCT3 were the same.
The graphs of the upper stage show the results of analysis of SSEA4 and OCT4 expressions, where the left of each set indicates the results of staining with a control antibody and the right of each set indicates the results of staining with a target antibody (anti-SSEA4 antibody or anti-OCT4 antibody). The data of staining with the target antibodies shifted upwards with increments in FL2 values compared to those of staining with the control antibody. Thus, expressions of the target proteins were detected in the majority of the cells. The graphs of the lower stage illustrate the results shown in graphs of the upper stage as histograms. It is obvious that the distribution curves obtained with the target antibodies shifted rightwards with increments in FL2 values compared to those with the control antibody.

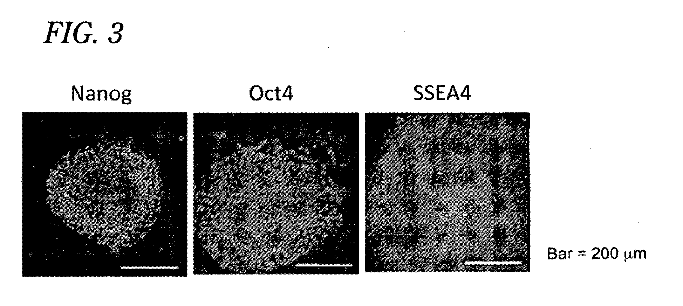

Furthermore, expressions of undifferentiation markers of human pluripotent stem cells, SSEA4, OCT3/4, and Nanog, were also confirmed by immunostaining.
Specifically, SeV-iPS cells prepared in Example 1 were subjected to fixation with acetone/methanol (1:3) and cell membrane permeabilization with 0.1% Triton-X-100/PBS, followed by a primary antibody reaction using an anti-human SSEA4 antibody (ES Cell Marker Sample Kit) (Millipore Co., Bedford, MA, USA), anti-human OCT3/4 antibody (ES Cell Marker Sample Kit) (Millipore Co.), or anti-human Nanog antibody (ReproCELL Inc., Tokyo, Japan) diluted to 1/100. The reactions with the anti-human SSEA4 antibody and the anti-human OCT3/4 antibody were performed in accordance with the protocols attached to the kits. Then, a secondary antibody reaction was performed using an Alexa Fluor 488-labeled anti-rabbit IgG antibody (Life Technologies, Inc.) diluted to 1/2000, followed by observation with a fluorescence microscope.
As shown in the results of immunostaining shown in Fig. 3, it was confirmed that the SeV-iPS cells prepared in Example 1 highly express the undifferentiation markers, SSEA4, OCT4, and Nanog. The results regarding OCT3 were the same.

### [Example 3] Removal of SeV vector-derived foreign gene

SeV vector-derived foreign genes were removed from SeV-iPS cells prepared in Example 1, followed by cloning to obtain a cell line.
As a reference of removal of SeV vector-derived foreign genes, immunostaining with an anti-SeV antibody (Medical & Biological Laboratories Co., Ltd., Nagoya, Japan) was performed. SeV-iPS cells were fixed with 10% Mildform (WAKO Pure Chemical Industries, Ltd., Osaka, Japan) and were stained using an anti-SeV antibody as a primary antibody and an Alexa Fluor 488-labeled anti-rabbit IgG antibody (Life Technologies, Inc.) as a secondary antibody, followed by observation with a fluorescence microscope.

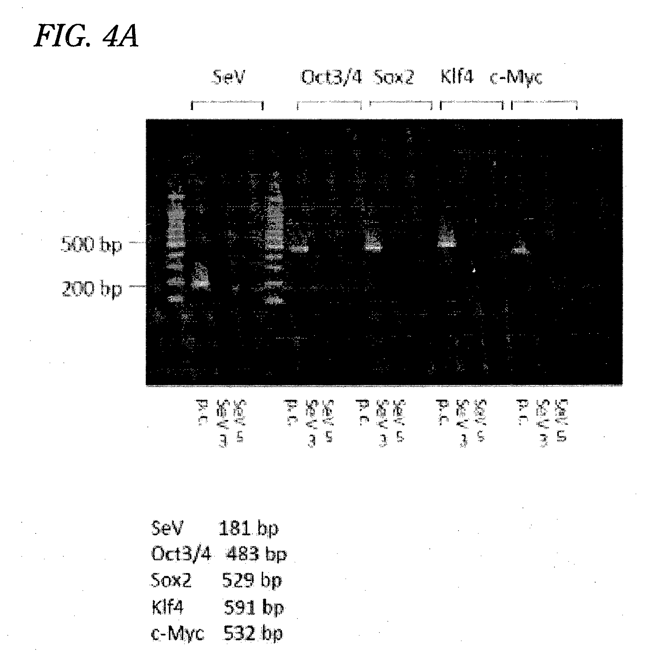

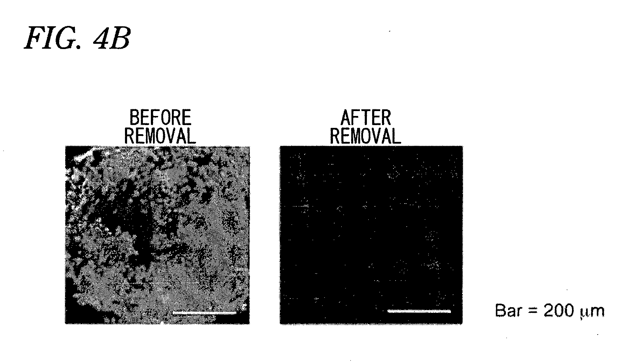

Furthermore, transgenes and SeV genomes were detected by reverse transcription-polymerase chain reaction (RT-PCR). The RT was performed using Superscript III First-Strand Synthesis System for RT-PCR (Life Technologies, Inc.), and the PCR was performed using GeneAmpR PCR System 9700 (Life Technologies, Inc.) with denaturation (at 94°C for 5 min), amplification (30 to 35 cycles of at 94°C for 30 seconds, at 55°C for 30 seconds, and at 72°C for 30 seconds), and post-extension (at 72°C for 10 min). The primers used were as follows:
OCT3/4 (Fw: CCCGAAAGAGAAAGCGAACCAG, Rv: AATGTATCGAAGGTGCTCAA),
SOX2 (Fw: ACAAGAGAAAAAACATGTATGG, Rv: ATGCGCTGGTTCACGCCCGCGCCCAGG),
KLF4 (Fw: ACAAGAGAAAAAACATGTATGG, Rv: CGCGCTGGCAGGGCCGCTGCTCGAC),
cMYC (Fw: TAACTGACTAGCAGGCTTGTCG, Rv: TCCACATACAGTCCTGGATGATGATG), and
SeV (Fw: GGATCACTAGGTGATATCGAGC, Rv: ACCAGACAAGAGTTTAAGAGATATGTATC).
As shown in the results of electrophoresis shown in Fig. 4A and the microscopic photographs in Fig. 4B, it was confirmed that the SeV-iPS cells prepared in Example 1 are SeV antigen negative and therefore do not hold any SeV vector-derived foreign genes. Accordingly, the SeV-iPS cell line is suitable for clinical use and also use in a drug evaluation system or disease model system, compared to iPS cells produced with a retrovirus vector.

### [Example 4] Culture for maintaining human embryonic stem cell and human induced pluripotent stem cell

The human embryonic stem cells (KhES-1) were supplied by Institute for Frontier Medical Sciences, Kyoto University. The human induced pluripotent stem cells produced using retrovirus vectors were supplied by Center for iPS Cell Research and Application, Kyoto University (253G1) and National Center for Child Health and Development (#40). KhES-1, 253G1, #40, and SeV-iPS cell lines were subjected to maintenance culture on a MEF layer irradiated with X-ray using a medium containing a 20% Knockout Serum Replacement (KSR) (Life Technologies, Inc.), 5 ng/mL FGF 2, 1% non-essential amino acid solution, 100 µM 2-mercaptoethanol, and 2 mM L-glutamine-containing DMEM/F12 (Life Technologies, Inc.).

### [Example 5] Induction of hepatocytes from human embryonic stem cell and human induced pluripotent stem cell

Before differentiation into hepatocytes, suspensions of pluripotent stem cells collected by treatment with a dissociation solution for separating and removing the MEF from the KhES-1, 253G1, #40, and SeV-iPS cell lines was left to stand in a centrifuge tube for about 30 seconds to selectively precipitate only the pluripotent stem cells, and the pluripotent stem cells were cultured on a culture plate coated with Matrigel (BD Biosciences). It is important on this occasion that the pluripotent stem cells are seeded at such a high density that there is no free area exposed on the Matrigel-coated plate where the cells are not attached.

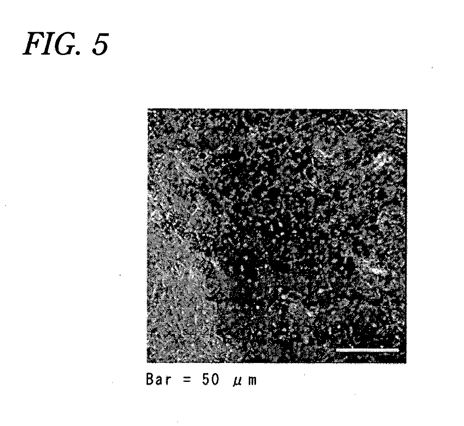

Induction of differentiation into hepatocytes was performed by the following five steps:
(1) Culture in a RPMI medium (Invitrogen Corporation) containing 100 ng/mL activin A, 25 ng/mL Wnt3A, and human recombinant albumin for 24 hours,
(2) Culture in a RPMI medium containing 100 ng/mL activin A and 0.2% KSR for 24 hours,
(3) Culture in a RPMI medium containing 10 ng/mL FGF 2, 20 ng/mL BMP 4, 200 ng/mL SHH, and 2% KSR for 5 days,
(4) Culture in a RPMI medium containing 10 ng/mL FGF 2, 20 ng/mL BMP 4, 20 ng/mL HGF, and 2% KSR for 5 days, and
(5) Culture in a RPMI medium containing 10 ng/mL oncostatin M, 0.1 µM dexamethasone, and 2% KSR for 6 to 16 days.
The culture through these five steps gave hepatocyte populations containing three-dimensional constructions characteristic to endoderm-derived tissue and polygonal structures characteristic to hepatocyte culture from the KhES-1, 253G1, #40, and SeV-iPS cells, as shown in the microscopic photograph in Fig. 5. Fig. 5 shows a phase contrast microscopic photograph of #40 as a typical example. Similar results were obtained in KhES-1, 253G1, and SeV-iPS cells.

### [Example 6] Evaluation by RT-PCR of hepatocyte induced from human induced pluripotent stem cell

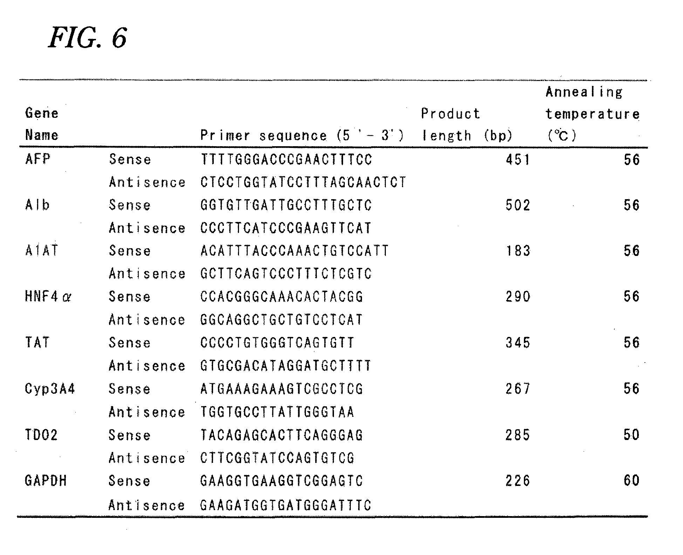

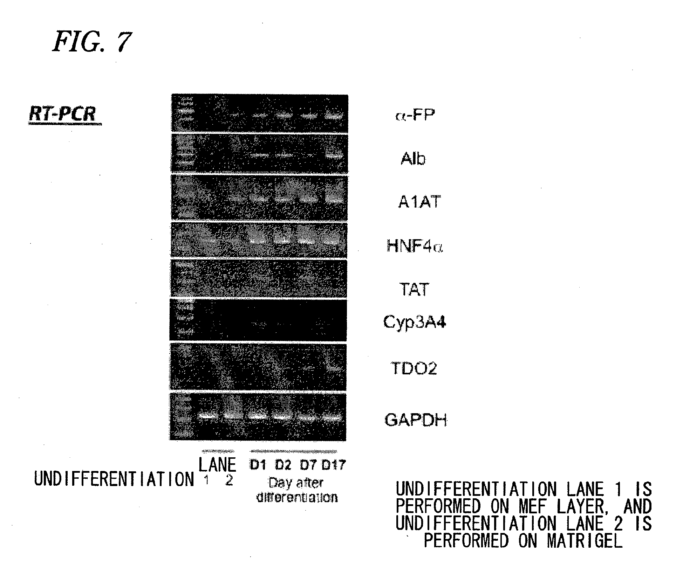

Total RNAs were extracted from human induced pluripotent stem cells (253G1) in the undifferentiated state and in the hepatocyte differentiation induced state using TRIzol (LifeTechnologies, Inc.), followed by RT-PCR using 1 µg of obtained total RNA. PCR was performed with denaturation (at 94°C for 5 min), amplification (35 cycles of at 94°C for 1 min, at 50 to 60°C for 1 min, and at 72°C for 30 seconds), and post-extension (at 72°C for 10 min). The primers used are shown in Fig. 6.
As shown in the results of electrophoresis shown in Fig. 7, it was confirmed that in the hepatocytes induced from human induced pluripotent stem cells, the gene expressions of α-FP, albumin, A1AT, HNF4α, TAT, CYP3A4, and TDO2 were induced over time after differentiation induction.

### [Example 7] Evaluation by real-time RT-PCR of hepatocyte induced from human induced pluripotent stem cell

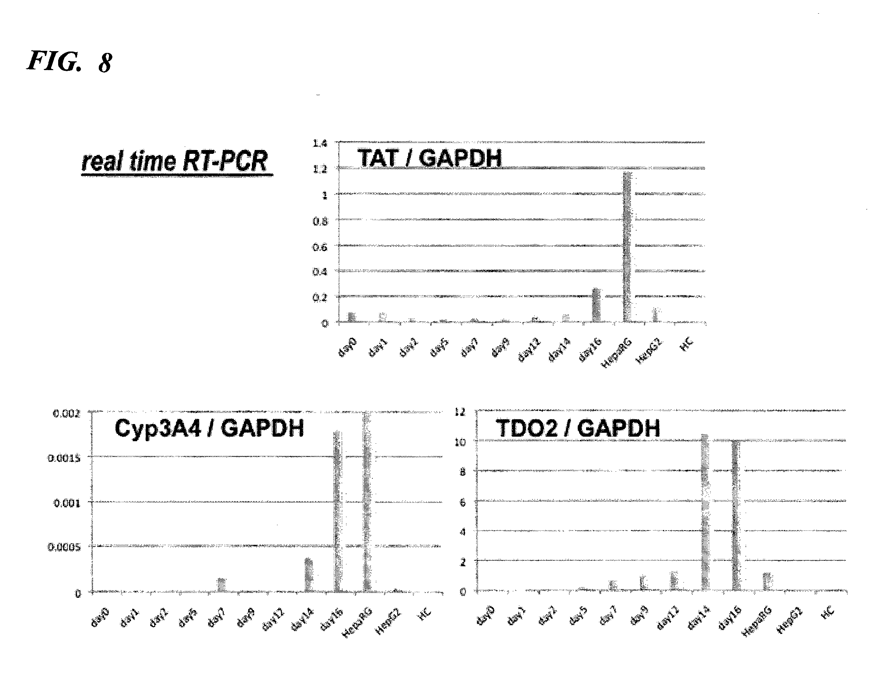

Total RNA for real-time RT-PCR was extracted using TRIzol (Life Technologies, Inc.) from all cells on the culture plate used for inducing differentiation into hepatocytes, followed by RT using 1 µg of the total RNA to synthesize cDNA. The cDNA was subjected to duplex real-time RT-PCR by ABI PRISM 7900 HT sequence Detection System (Life Technologies, Inc.) using TaqMan target probe/primer, GAPDH probe/primer, and QuantiTect Multiplex PCR Master Mix (Qiagen Science, Maryland, USA). Amplification was performed, after a reaction at 50°C for 2 min and at 95°C for 10 min, by 45 cycles of a reaction at 95°C for 15 seconds and at 60°C for 1 min. The results were standardized to GAPDH. The probes/primers were TD02 (Assay ID: Hs00194611_ml), Cyp3A4 (Assay ID: Hs01546612_ml), and TAT (Assay ID: Hs00356930_ml).
As shown in the graphs of Fig. 8, it was observed that in the hepatocytes induced from human induced pluripotent stem cells (253G1), expressions of genes of TDO2, CYP3A4, and TAT increase with maturation of the hepatocytes. The increases in expression of TDO2 and TAT were about 2000 times and 2.8 times, respectively, those of undifferentiated cells on the 16th day after the differentiation induction. The increase in expression of Cyp3A4 was about 566 times that of HepG2 on the 16th day after the differentiation induction.

### [Example 8] Evaluation by immunostaining of hepatocyte induced from human embryonic stem cell

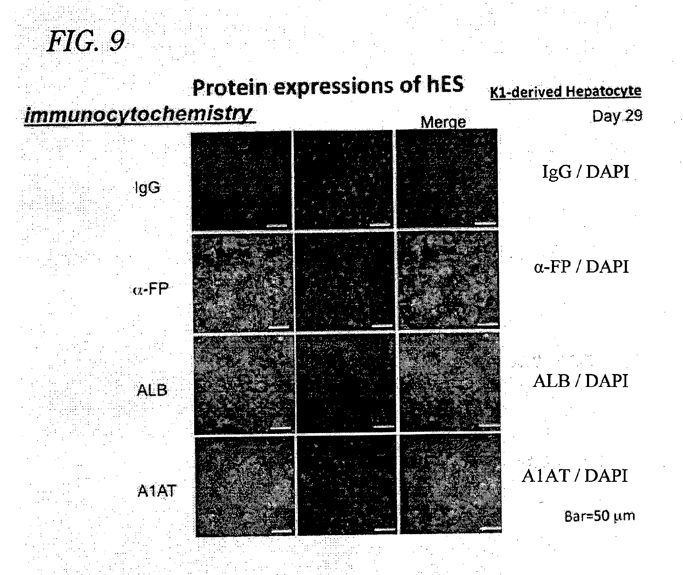

Cells were subjected to fixation with acetone/methanol (1:3) and cell membrane permeabilization with 0.1% Triton-X-100/PBS, followed by a primary antibody reaction using an anti-human α-fetoprotein (α-FP) antibody (Epitomics Inc., Burlingame, CA, USA) diluted to 1/500, anti-albumin antibody (DAKO A/S, Denmark), anti-human α1-antitrypsin (A1AT) antibody (Lifespan Bioscience, Inc., Seattle, WA) diluted to 1/500, or anti-human cytochrome P450 (Cyp) antibody (Abcam Plc, Cambridge, UK) diluted to 1/2000. Then, a secondary antibody reaction was performed using an Alexa Fluor 488-labeled anti-rabbit IgG antibody (Life Technologies, Inc.) diluted to 1/2000, followed by observation with a fluorescence microscope.
As shown in the results of immunostaining shown in Fig. 9, signals of α-FP, albumin, and A1AT were detected in the hepatocytes induced from human embryonic stem cells.
In Fig. 9, the image stained with an antibody against the target protein is shown in green, and the image of a nucleus stained with DAPI is shown in blue. In all cells of which nuclei were stained, the cytoplasm was stained in green. That is, all cells were stained, and the intracellular localization of the target protein and the presence of individual cells were clarified. In Fig. 9, "K1" of K1-derived Hepatocyte indicates a human ES cell line KhES-1 established at Kyoto University.

### [Example 9] Evaluation by Western blotting of hepatocytes induced from human embryonic stem cell and human induced pluripotent stem cell

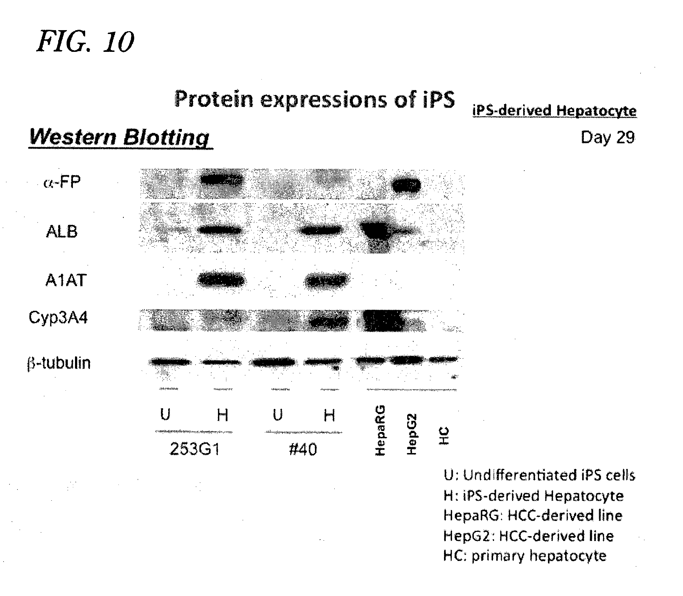

A solution of differentiation induced cells was mixed with the same amount of a sample buffer for SDS-PAGE, followed by thermal denaturation at 98°C for 5 minutes. SDS-PAGE on a 12% acrylamide gel and transfer to a PVDF membrane by semi-dry blotting were performed. Blocking with 5% milk/TBS-T was performed, and a primary antibody reaction was performed using an anti-α-FP antibody (Epitomics Inc.) diluted to 1/500, anti-albumin antibody (DAKO A/S) diluted to 1/20000, anti-A1AT antibody (Lifespan Bioscience) diluted to 1/500, or anti-Cyp antibody (Abcam Plc) diluted to 1/2000. After a secondary antibody reaction using a HRP-labeled anti-rabbit IgG (Cell Signaling Technology, Inc., Beverly, MA, USA) diluted to 1/2000, the signal was exposed to an X-ray film (FUJIFILM, RX-U) (Fuji Photo Film Co., Ltd., Tokyo, Japan) using an ECL Western blotting detection system (GE Healthcare, Fairfield, CT, USA).
As a result, expressions of proteins characteristic to hepatocytes were detected in hepatocytes induced from human embryonic stem cells and human induced pluripotent stem cells. Fig. 10 shows the results of Western Blotting of KhES-1 as a typical example. Expressions of proteins, α-FP, albumin, A1AT, and CYP3A4, characteristic to hepatocytes were detected. The primary cultured hepatocytes (HCs) were subcultured for a minimum number of passages after acquisition to obtain sufficient numbers of cells required for the experiments, and, as a result, the expressions of functional proteins disappeared. In the figure, 253G1 refers to a human iPS cell line established at Kyoto University, and #40 refers to a human iPS cell line established at National Center for Child Health and Development.

### [Example 10] Evaluation by PAS staining of hepatocytes induced from human embryonic stem cell and human induced pluripotent stem cell by PAS staining

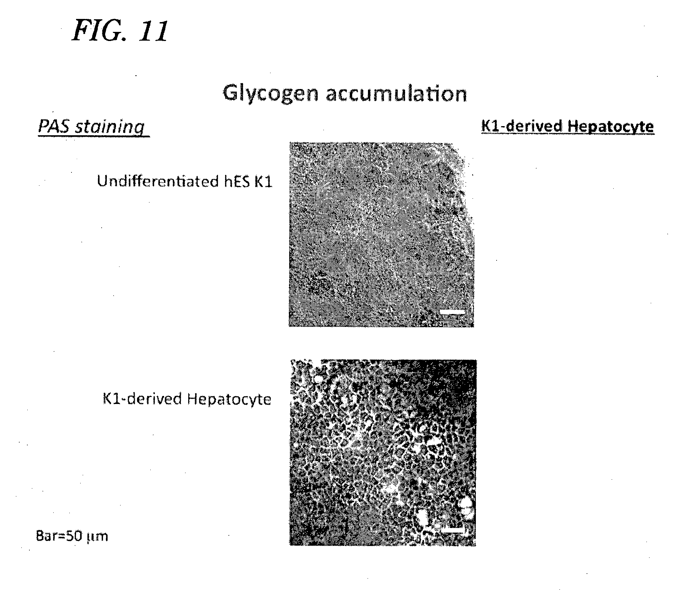

Glycogen in hepatocytes was detected using a Periodic Acid Schiff (PAS) staining kit (Muto Pure Chemicals Co. Ltd., Tokyo).
Hepatocytes induced from human embryonic stem cells and human induced pluripotent stem cells were fixed using 10.5% formaldehyde at room temperature and were treated with 1% periodic acid for 10 minutes, followed by Schiff staining at 37°C for 30 minutes. After counter staining with hematoxyline, observation under a microscope was performed.
As a result, PAS staining positive cells were observed at a high rate in the hepatocytes induced from human embryonic stem cells and human induced pluripotent stem cells. Fig. 11 shows the results of PAS staining of KhES-1 as a typical example. In the figure, both of K1 of K1-derived Hepatocyte and hES K1 of Undifferentiated hES K1 refer to a human ES cell line, KhES-1, established at Kyoto University.

### [Example 11] Evaluation of ICG-uptaking and releasing abilities of hepatocytes produced from human embryonic stem cell and human induced pluripotent stem cell

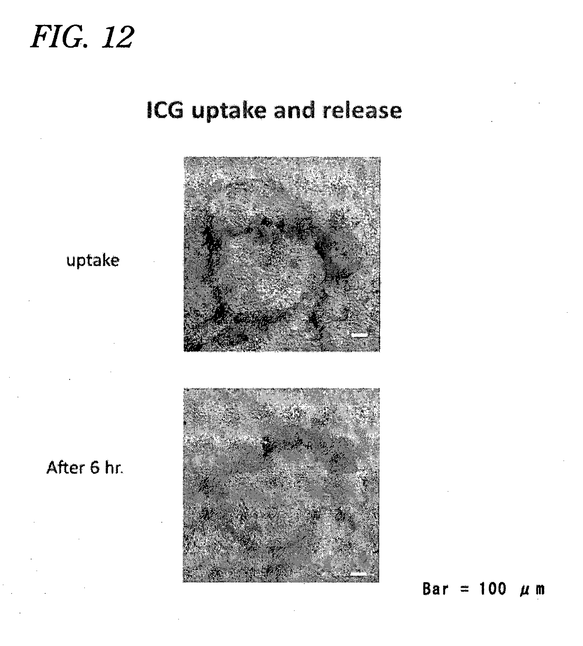

The detoxication ability (ability of processing foreign substances) of hepatocytes was measured through ICG uptake and release.
The ICG uptake was evaluated by incubating hepatocytes with 1 mg/mL of ICG at 37°C for 30 minutes, washing the hepatocytes with PBS, and observing the hepatocytes under an optical microscope. The ICG release was evaluated by further culturing the hepatocytes in an ICG-free medium for 6 hours and then observing the hepatocytes under an optical microscope.
As a result, ICG uptake and release were confirmed in hepatocytes produced from human embryonic stem cells and human induced pluripotent stem cells. Fig. 12 indicates the micrographs of KhES-1 as a typical example. In the figure, ICG is shown in green. In the photograph of the upper stage, there are cells stained is in green due to uptake of ICG, and in the photograph, of the lower stage, of the cell after culture for 6 hour culture, the ICG is excreted to the outside of the cell.

### [Example 12] Evaluation of CYP3A4 activities of hepatocytes induced from human embryonic stem cell and human induced pluripotent stem cell

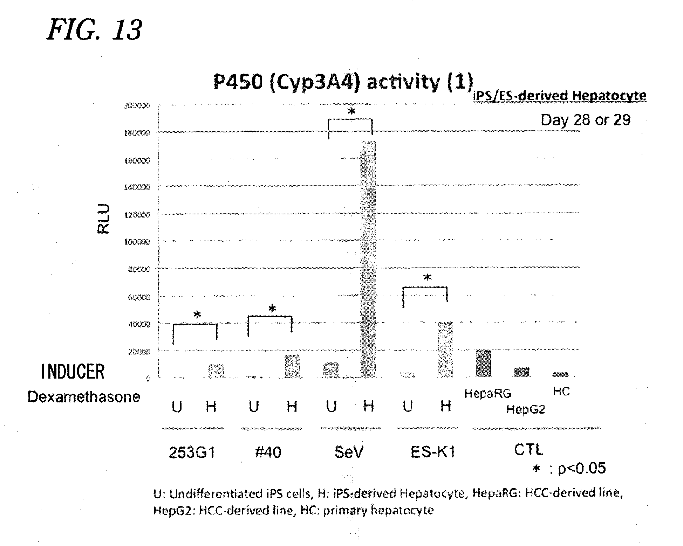

The cytochrome P450 3A4 (CYP3A4) activity of hepatocytes was measured with a p450-GloTM CYP3A4 measurement kit (Promega Co., Madison, WI, USA).
A synthetic substrate of CYP3A4 was added to the culture supernatant of hepatocytes stimulated with 50 µM dexamethasone or 100 µM Rifampicin for 16 hours. After 4 hours, the culture supernatant was collected, and the emission intensity was measured with a weak luminescence measuring apparatus (luminometer).
As shown in the graph of Fig. 13, CYP3A4 activities were detected in hepatocytes produced from human embryonic stem cells and human induced pluripotent stem cells. The activity levels were all several times or more higher than those of their undifferentiated counterparts in all cases. In the hepatocytes produced from human induced pluripotent stem cells that were established using a Sendai virus vector, the activity was eight or more times higher than that of HepaRG cells (BIOPREDIC International, Rennes, France).
The HepaRG cells were cultured in accordance with the protocol of BIOPREDIC international. The HepG2 cells were purchased from Health Science Research Resources Bank, and the human primary cultured adult hepatocyte cell line was purchased from DS Pharma Biomedical Co., Ltd. (Osaka, Japan). They were cultured using a CS-C medium kit of Cell Systems Corporation (Kirkland, WA, USA). In the figure, 253G1 refers to a human iPS cell line established at Kyoto University, #40 refers to a human iPS cell line established at National Center for Child Health and Development, SeV refers to an iPS cell line induced using a SeV vector, ES-K1 refers to a human ES cell line established at Kyoto University, and CTL is an abbreviation of control.

### [Example 13] Evaluation by electron microscopic photograph of hepatocytes induced from human embryonic stem cell and human induced pluripotent stem cell

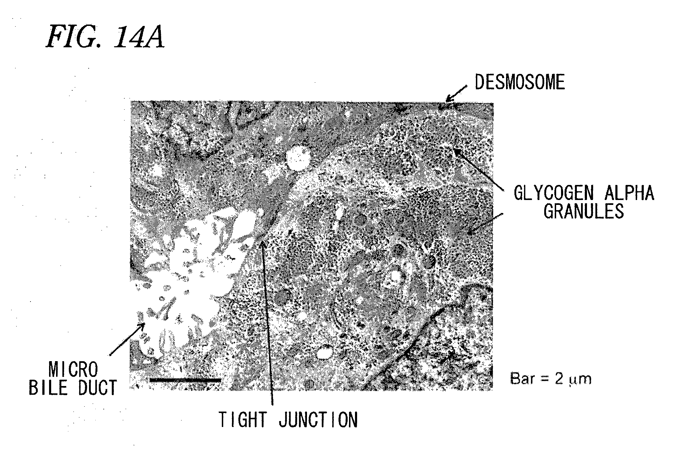

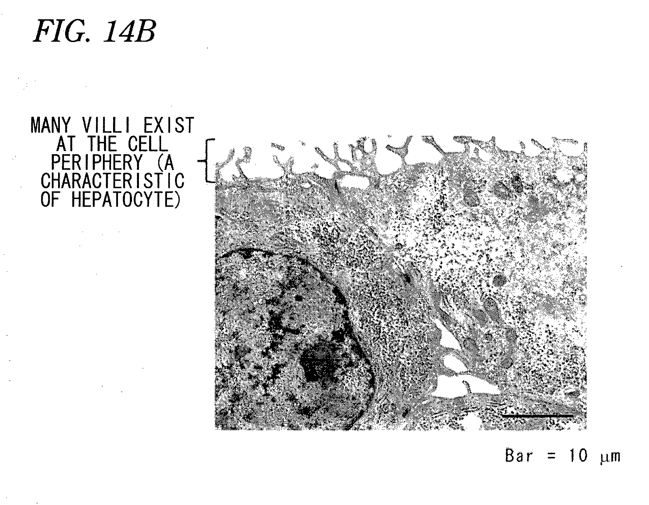

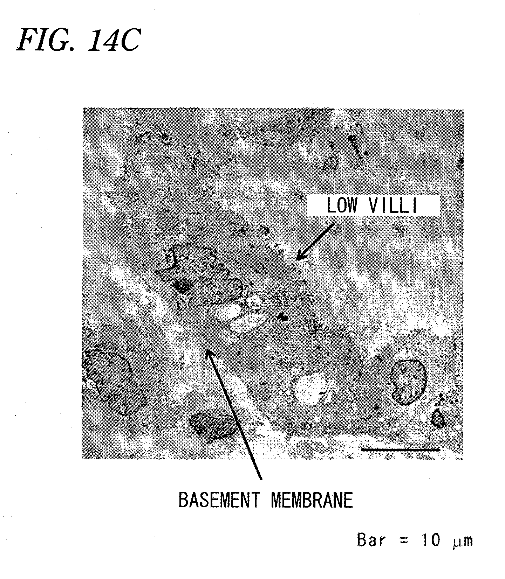

Pluripotent stem cell-derived hepatocytes were fixed with a 2.5% glutaraldehyde solution and then fixed with a 2% osmium tetraoxide solution at BML Inc. (Tokyo, Japan) and were embedded in Epon Resin. Slices of a thickness of 70 nm were prepared using an ultramicrotome and were observed under an electron microscope.
As shown in Figs. 14(A) and 14(B), cells rich in villi characteristic to hepatocyte were detected. In such cells, a structure specific to hepatocyte, i.e., cytoplasm glycogen α granules (rosette-forming glycogen granules), and a bile canaliculus at an intercellular boundary, was seeled by an adjacent tight junction and a desmosome were detected. Thus, production of mature hepatocytes was confirmed. As a typical example, the results of KhES-1 are shown.
Furthermore, as shown in Fig. 14(C), a structure specific to bile duct epithelial cell, i.e., short villi (villi that are obviously shorter than brush borders observed in renal tubules, small intestine, and large intestine and are characteristic to bile duct epithelial cell) on one side of a cell and a basement membrane on the other side, was detected. Thus, production of mature bile duct epithelial cells was confirmed. As a typical example, the results of #40 are shown.
Human embryonic stem cell-derived bile duct epithelial cells and human induced pluripotent stem cell-derived bile duct epithelial cells gave similar results.

### [Example 14] Evaluation of toxicity of drug using hepatocytes induced from human embryonic stem cell and human induced pluripotent stem cell

D-Galactosamine (D-GalN), which is known to have toxicity to hepatocytes, was added to pluripotent stem cell-derived hepatocytes at a final concentration of 25 mM, followed by culture at 37°C. After 24 hours, 1.5 mL of culture supernatant was collected and was centrifuged at 15000 rpm at 4°C to completely remove cell components. The concentrations of liver-derived deviation enzymes contained in the supernatant, i.e., glutamic oxaloacetic transaminase (GOT), glutamic pyruvic transaminase (GPT), γ-glutamyl transpeptidase (γ-GTP), leucine aminopeptidase (LAP), lactate dehydrogenase (LDH), and LDH isozyme (LDH 1 to 5), were measured (performed at BML Inc., Shibuya, Tokyo, Japan).
As shown in Fig. 15(A), in hepatocytes (28th day from differentiation induction) derived from human ES cells (KhES-1 cell line), the level of GOT, which is a mature hepatocyte deviation enzyme, increased depending on D-GalN administration. The levels of γ-GTP, which is a deviation enzyme from bile canaliculus (hepatocyte) and bile duct epithelial cells, and LAP, which is a deviation enzyme from bile duct epithelial cells, also increased. The level of LDH, which is a deviation enzyme from mature hepatocytes, also increased, and isozyme measurement thereof showed a "liver damage-type" pattern in which type 4 and type 5 are dominant. In contrast, in undifferentiated ES cells, as shown in Fig. 15(B), administration of D-GalN did not increase the levels of all of the deviation enzymes.

Hepatocytes derived human iPS cells (#40) also gave similar results. That is, as shown in Fig. 15(C), the levels of GOT and GPT, which are mature hepatocyte deviation enzymes, γ-GTP, which is a deviation enzyme from bile canaliculus (hepatocyte) and bile duct epithelial cells, and LAP, which is a deviation enzyme from bile duct epithelial cells, increased depending on D-GalN administration. In addition, LDH, which is a deviation enzyme from mature hepatocytes, also increased, and isozyme measurement thereof showed a "liver damage-type" pattern in which type 4 and type 5 are dominant. In contrast, in undifferentiated ES cells, as shown in Fig. 15(D), administration of D-GalN did not increase the levels of GOT, GPT, γ-GTP, LAP, and LDH.

Also in hepatocytes derived from SeV-iPS cells, similar results were obtained. That is, as shown in Fig. 15(E), when SeV-iPS-derived hepatocytes were cultured in the presence of D-GalN, the level of GOT in the culture supernatant increased. In contrast, as shown in Fig. 15(F), when undifferentiated SeV-iPS cells were cultured in the presence of D-GalN, the level of GOT in the culture supernatant did not increase.

When HepaRG cells, a commercially available hepatocyte cell line, were cultured in the presence of D-GalN, as shown in Fig. 15(G), the levels of GOT, GPT, LAP, and LDH-5 in the culture supernatants increased, but the level of γ-GTP did not increase. In addition, as shown in Fig. 15(H), when HepG2 cells were cultured in the presence of D-GalN, the levels of GOT, GPT, and LDH-5 increased, but the levels of γ-GTP and LAP did not increase.

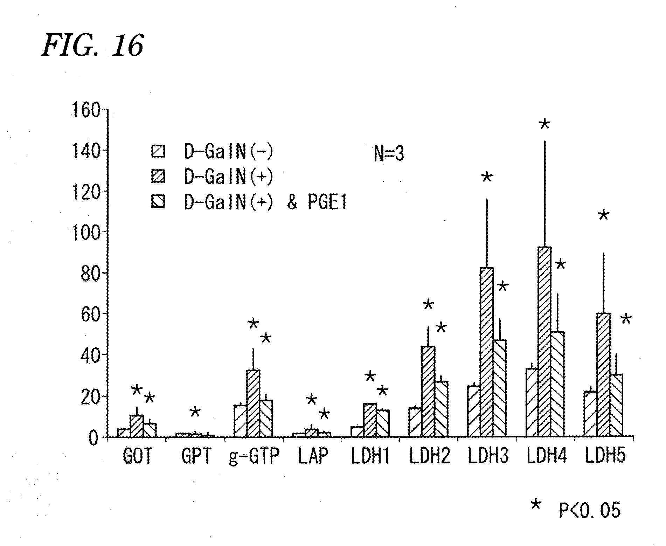

Furthermore, it was confirmed by the following experiment using prostaglandin E1 (PGE1) that the increases of the levels of deviation enzymes from the liver in culture supernatants, detected in the experiments shown in (A) to (H) of Fig. 15, are certainly caused by the toxicity of D-GalN on hepatocytes/bile duct epithelial cells. That is, 4 mM PGE1 was added to hepatocytes derived from a #40 cell line in advance, and the hepatocytes were cultured at 37°C for 4 hours and were subjected to a D-GalN administration experiment as in above. As a result, as shown in Fig. 16, the levels of all the deviation enzymes from the liver detected in the culture supernatant were significantly suppressed by pretreatment with PGE1. Similar tendencies were detected in the samples of hepatocyte differentiation induction from other human iPS cell lines and human ES cell lines.
PGE1 is known to have a hepatoprotective acitivity and a liver regeneration-promoting effect and is also reported to be effective, in also clinical, for inhibiting ischemia-reperfusion injury in hepatectomy or liver transplantation, improving the liver function after hepatectomy, and preventing mono organ disseminated intravascular coagulation in ABO incompatible liver transplantation.

As described above, it was demonstrated that hepatocyte enzymes deviate in the culture supernatant at levels that can be detected by generic biochemical examination technologies by culturing pluripotent stem cell-derived hepatocytes in the presence of a hepatotoxic drug. This method does not require labors such as collection of cells as in the cases of conventional methods but enables fast evaluations of hepatotoxicity quantitatively and extensively by merely collecting only about 1.5 mL of the culture supernatant. One of the greatest advantages of the evaluation system using pluripotent stem cell-derived hepatocytes according to the present invention is that the system has enabled detecting increments in the levels of both γ-GTP, which is a deviation enzyme from the bile canaliculus, and LAP, which is a deviation enzyme from bile duct epithelial cells. In the assays using existing cells such as HepaRG cells and HepG2 cells, simultaneous detections of the increments in levels of these deviation enzymes has not been achieved. Therefore, in order to exhaustively evaluate hepatotoxicity (cytoplasm of hepatocyte: GOT and LDH, cell membrane of hepatocyte (bile canaliculus): γ-GTP, and bile duct epithelial cells: LAP), the use of pluripotent stem cell-derived hepatocytes is indispensable.

### [Example 15] Identification of hepatic stem/progenitor cell populations in hepatocytes derived from human embryonic stem cell and human induced pluripotent stem cell

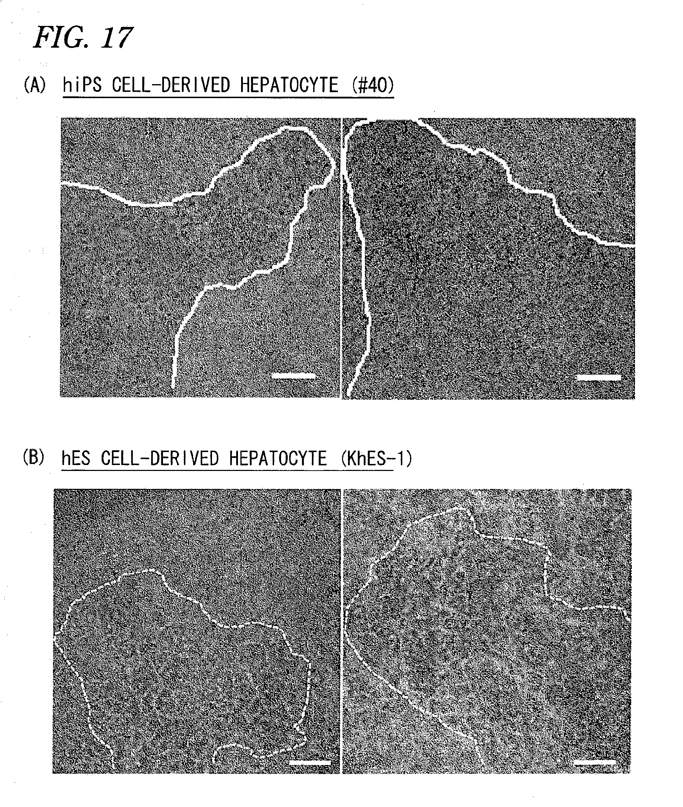

Pluripotent stem cell-derived hepatocytes contain both a two-dimensional region, as shown in Fig. 5, where polygonal cell populations planarly spread, and a three-dimensional construction region raised by multilayered cells (Fig. 17). Morphologically, the former shows a characteristic of mature hepatocyte, and the latter shows a characteristic of hepatic stem/progenitor cell. It is therefore strongly suggested that pluripotent stem cell-derived cells include hepatic stem/progenitor cells, which are present in the three-dimensional construction region.
In order to verify the above and to establish a simple method of identifying and separating hepatic stem/progenitor cells by morphological observation, the expressions of epithelial cell adhesion molecule (EpCAM) and α-FP, which are markers of hepatic stem/progenitor cells, were investigated by immunostaining. Fig. 18(A) shows the results of immunostaining of EpCAM and α-FP in hepatocytes (28th days after differentiation induction) produced from human induced pluripotent stem cells (#40). As shown in the figure, EpCAM and α-FP were expressed in agreement with the three-dimensional construction region. The cells in the three-dimensional construction region were in the proliferative cycle, which was confirmed by an uptake test of a nucleic acid analog, bromodeoxyuridine (BrdU), (the left in Fig. 18(B)) and immunostaining of a proliferative marker, Ki67, (the right in Fig. 18(B)). In the two-dimensional region where polygonal cell populations planarly spread, the expressions of mature hepatocyte markers, Alb (Fig. 18(C)) and AAT (Fig. 18(D)), were detected by immunostaining. Those findings were further confirmed in the cases of other human induced pluripotent stem cell lines (Fig. 18(E), (F)).
Thus, it was revealed that in the hepatocyte differentiation induction products from pluripotent stem cells, the cell populations in the three-dimensional construction region consist of hepatic stem/progenitor cells, and the cell populations in the two-dimensional region consist of mature hepatocytes.

### [Example 16] Subculture of hepatic stem/progenitor cell populations in hepatocytes induced from human embryonic stem cell and human induced pluripotent stem cell

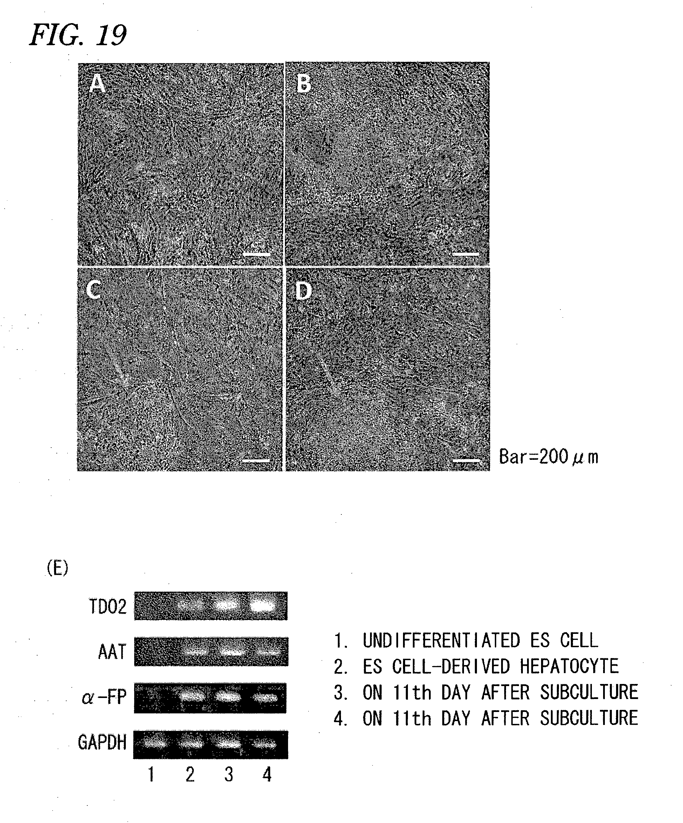

The hepatic stem/progenitor cell populations (cell populations forming the three-dimensional construction in the hepatocyte differentiation induction products) derived from pluripotent stem cells identified in the prior Example were collected and were subjected to subculture. First, a method for cell detachment to collect cell populations was investigated. It was revealed that detachment (separation) of cell populations forming a three-dimensional construction region with a protease such as trypsin or collagenase or a solution containing a chelating agent such as a divalent ion considerably reduced cell viability of the cells and that almost all cells died after subculture. Accordingly, the three-dimensional construction region was mechanically cut out with a micro knife, and after disaggregation by gentle pipetting, cells were then transferred onto a fresh culture plate coated with Matrigel (manufactured by Becton, Dickinson and Company), and were cultured at 37°C in a 5% by volume carbon dioxide gas incubator.
As a result, as shown in Fig. 19, the subcultured cell populations remained viable and started to proliferate. On the 11th day of the subculture, cells had spread all over the culture plate (Fig. 19(A)) and a three-dimensional constructions were again confirmed therein (Fig. 19(B), the position indicated with an arrow). In other areas of three-dimensional construction regions, a cell population with morphologies very similar to those of hepatocyte progenitor cells called small hepatocyte colonies (Non-Patent Literatures 11 to 12) was also detected (Fig. 19(C), (D), the positions indicated with arrows).

The cell populations proliferated by subculture were subjected to an investigation by RT-PCR for the expressions of hepatocyte progenitor cell marker, α-FP, and mature hepatocyte markers, TDO2 and AAT. As a result, as shown in Fig. 19(E), expressions of α-FP and AAT were maintained after subculture, and the expression of TDO2 was rather higher than that before the subculture. These results indicated that the cell populations forming a three-dimensional construction region proliferate even after subculture with an increasing degree of maturity of hepatocytes, while maintaining the presence of liver progenitor cells.
As described above, it was demonstrated that the cell population forming the three-dimensional construction in the hepatocytic differentiation products of pluripotent stem cells is a hepatic stem/progenitor cell population and that the cell population can easily be passaged and expanded by subculture through mechanical detachment with, for example, a micro knife, subsequent disaggregation and further culture on a Matrigel-coated plate.
As a method of purifying hepatic stem/progenitor cells from a human pluripotent stem cell-derived cell population, a cell-sorting technology using an anti-N-cadherin antibody has been reported (Non-Patent Literature 13). This method, however, needs a staining step using a specific antibody and a step of fractionating cells using an expensive device such as a cell sorter and needs co-culture on a mouse-derived feeder cells thereafter (STO cell line) for proliferating the purified hepatic stem/progenitor cells. In contrast, in the method according to the present invention, hepatic stem/progenitor cells can be purified by a simple operation, i.e., collection of a three-dimensional construction region by, for example, using a micro knife under a phase contrast microscope. Moreover, there is an advantage in that purified hepatic stem/progenitor cells can be expanded by culture under feeder-free conditions (such as a plate coated with Matrigel).
Thus, the technology for purifying and subculturing pluripotent stem cell-derived hepatic stem/progenitor cells according to the present invention is highly versatile and is a feeder-free culture system that does not use any xenogenous animal cells at all and is therefore a method having notably high feasibility for producing pluripotent stem cell-derived hepatic stem/progenitor cells.

### Industrial Applicability

The present invention has overcome various problems associated with conventional drug metabolism/toxicity evaluation systems, i.e., problems such as instability in data, inter-lot differences, inter-cell-line differences, costs, and inability to determine individual differences, and thereby can provide an innovative tool for drug discovery research. In addition, the invention can contribute to safety examination of drugs in the preclinical study stage.
Human ES cells, human iPS cells, and the like as one of starting materials, from which hepatocytes that are applied to the drug metabolism/toxicity evaluation are produced according to the present invention, have infinite proliferation ability. It is therefore highly feasible to stably produce the hepatocytes at an industrial scale. In addition, the hepatocytes can be produced from human pluripotent stem cells within one month, and therefore a supply corresponding to a demand is also possible. Furthermore, the method of producing hepatocytes uses only generic cell culture facilities and therefore can be implemented in any country and area in all over the world. Accordingly, the method can be expanded to huge plant industry and is practical and has a high industrial value.

## Claims

1. A method of producing highly functional hepatocytes using pluripotent stem cells, the method comprising:
preparing pluripotent stem cell-derived primitive endoderms from pluripotent stem cells by a method including the steps (A) and (B);
preparing liver progenitor cells from the pluripotent stem cell-derived primitive endoderms by a method including the step (C); and
preparing highly functional hepatocytes from the liver progenitor cells by a method including the step (D),
(A) culture under serum-free and feeder-free conditions;
(B) culture in the presence of albumin and at least one cytokine;
(C) culture in the presence of SHH or an SHH agonist and at least one cytokine; and
(D) culture to promote maturity in the presence of at least one cytokine.

2. A method of producing pluripotent stem cell-derived primitive endoderms using pluripotent stem cells, the method comprising:
preparing pluripotent stem cell-derived primitive endoderms from pluripotent stem cells by a method including the steps (A) and (B),
(A) culture under serum-free and feeder-free conditions; and
(B) culture in the presence of albumin and at least one cytokine.

3. The method of producing pluripotent stem cell-derived primitive endoderms according to Claim 1 or 2, wherein
in the step (A),
the pluripotent stem cells are seeded at a density such that agglomerates of the cells are in contact with one another.

4. The method of producing pluripotent stem cell-derived primitive endoderms according to any one of Claims 1 to 3, wherein
in the step (A),
feeder cells are removed by separation utilizing a differentiation in sedimentation velocity.

5. The method of producing pluripotent stem cell-derived primitive endoderms according to any one of Claims 1 to 4, wherein
in the step (B),
the albumin is human recombinant albumin.

6. A method of producing liver progenitor cells using primitive endoderms, the method comprising:
preparing liver progenitor cells from primitive endoderms by a method including the step (C),
(C) culture in the presence of SHH or an SHH agonist and at least one cytokine.

7. The method of producing liver progenitor cells according to Claim 6, wherein
in the step (C),
the primitive endoderms are pluripotent stem cell-derived primitive endoderms.

8. A method of producing highly functional hepatocytes using liver progenitor cells, the method comprising:
preparing highly functional hepatocytes from liver progenitor cells by a method including the step (D),
(D) culture to promote maturity in the presence of at least one cytokine.

9. The method according to any one of Claims 1 to 8, wherein
pluripotent stem cells are ES cells or iPS cells.

10. The method according to Claim 9, wherein
the iPS cells are those established using a Sendai virus vector.

11. The method according to any one of Claims 1 to 10, wherein
the pluripotent stem cells are human pluripotent stem cells.

12. A highly functional hepatocyte produced using a pluripotent stem cell, wherein the pluripotent stem cell-derived highly functional hepatocyte is produced by
preparing pluripotent stem cell-derived primitive endoderms from pluripotent stem cells by a method including the steps (A) and (B);
preparing liver progenitor cells from the pluripotent stem cell-derived primitive endoderms by a method including the step (C); and
preparing highly functional hepatocytes from the liver progenitor cells by a method including the step (D),
(A) culture under serum-free and feeder-free conditions;
(B) culture in the presence of albumin and at least one cytokine;
(C) culture in the presence of SHH or an SHH agonist and at least one cytokine; and
(D) culture to promote maturity in the presence of at least one cytokine.

13. A highly functional hepatocyte produced from a pluripotent stem cell, wherein the pluripotent stem cell-derived highly functional hepatocyte shows a cytochrome P450 enzymatic activity in the presence of dexamethasone at a level twice or more that of a HepaRG cell, has ICG uptake and release activities, is PAS staining positive, expresses A1AT, and can also differentiate into a bile duct epithelial cell.

14. A pluripotent stem cell-derived primitive endoderm produced from a pluripotent stem cell, wherein the pluripotent stem cell-derived primitive endoderm is produced from pluripotent stem cells by a method including the steps (A) and (B):
(A) culture under serum-free and feeder-free conditions; and
(B) culture in the presence of albumin and at least one cytokine.

15. A liver progenitor cell produced using a primitive endoderm, wherein
liver progenitor cells are prepared from primitive endoderms by a method including the step (C):
(C) culture in the presence of SHH or an SHH agonist and at least one cytokine.

16. A highly functional hepatocyte produced using a liver progenitor cell, wherein
highly functional hepatocytes are prepared from liver progenitor cells by a method including the step (D):
(D) culture to promote maturity in the presence of at least one cytokine.

17. The pluripotent stem cell-derived highly functional hepatocyte or the pluripotent stem cell-derived primitive endoderm according to any one of Claims 12 to 14, wherein
the pluripotent stem cell is an ES cell or an iPS cell.

18. The pluripotent stem cell-derived highly functional hepatocyte or the pluripotent stem cell-derived primitive endoderm according to any one of Claims 12 to 17, wherein
the pluripotent stem cell is a human pluripotent stem cell.

19. A method of testing metabolism of a drug, the method comprising:
quantitatively measuring metabolism of a drug using pluripotent stem cell-derived highly functional hepatocytes according to any one of Claims 12 to 18.

20. A method of testing toxicity of a drug, the method comprising:
quantitatively measuring cell death caused by a drug using pluripotent stem cell-derived highly functional hepatocytes according to any one of Claims 12 to 18.
